# EUROPEAN PATENT APPLICATION

(11) **EP 4 442 221 A1**
(43) Date of publication of application: **09.10.2024**
(21) Application number: 24165778.2
(22) Date of filing: 25.03.2024
(51) Int. Cl.: A61B 50/36, B65F 1/16

(54) **CONTAINER WITH IMPROVED LID**

(30) Priority: 05.04.2023 IT 202300006705
(71) Applicant: Isi Plast S.p.a., 42015 Correggio (Reggio Emilia) (IT)
(72) Inventor: MELLI, Gianluca, 42015 CORREGGIO (RE) (IT); DAVOLI, Giacomo, 42015 CORREGGIO (RE) (IT)
(74) Representative: Studio Torta S.p.A.

(57) **Abstract**

A container with a permanent closure having a box body (2) and a cover (3), which are configured to interlock with each other in an indissoluble way; wherein the box body (2) comprises a collar (8) of polygonal shape with rounded corners having both straight sides and corners which connect two adjacent straight sides to each other; wherein the collar (8) is integral, in other words it has no through openings, in correspondence of the corners; wherein the collar (8) of the box body (2) comprises a projecting part (15) disposed in correspondence of a respective corner (11); wherein the projecting part (15) and the flap (24) are configured to form an interference coupling.

## Description

### CROSS-REFERENCE TO RELATED APPLICATIONS

This patent application claims priority from Italian patent application no. 102023000006705 filed on April 5, 2023, the entire disclosure of which in incorporated herein by reference.

### TECHNICAL FIELD

This patent application relates to an improved container with a cover, in particular containers with a cover with permanent closure.

### BACKGROUND ART

For hospital use, it is known to use containers with a cover with permanent closure for storing, transporting and incinerating potentially infectious hospital waste. In particular, containers are known with a cover, which once closed, forms with a containing box body an indivisible shape and interference coupling. In this way, the container is substantially sealed, making it impossible for potentially infectious waste to escape unless the cover and/or the container itself are broken. Containers with a cover of the type described above must have meet the requirements of resistance to external stress dictated by ISO standards and which determine the level of strength of the container when, for example, subjected to phenomena such as falling from a height.

Different types of cover are known to allow the connection between the cover and the box body to be implemented. For example, the production is known of containers having a cup-shaped body having an inner housing accessible through a collar which has a plurality of openings. The cover of the container is configured to be fitted over the collar and comprises a plurality of snap-fit couplings, each of which is configured to be inserted inside a respective opening and to fasten, for example by means of a bayonet system, to the collar itself. Generally, the openings and the snap-fit couplings are produced along the straight margins of the collar and, respectively, of the cover. Solutions of this type can have the drawback of not all the couplings being correctly activated. This can facilitate the disadvantage of undesirable opening of the container. Moreover, in the prior art solutions, once the cover has been partially coupled, it is virtually impossible to intervene in order to adjust it and ensure that it closes correctly. Moreover, the closure of the cover of these prior art solutions has the disadvantage of having limited resistance if dropped.

The document EP3194287 B1 discloses a container having, in correspondence of the four corners of the collar, latching openings that are curved or produced along inclined portions. According to the teachings of EP3194287 B1, the cover comprises, in correspondence of the four corners, snap-fit couplings of a shape corresponding to the shape of the latching opening. The particular shape of the latching openings and of the relevant snap-fit couplings requires the cover to be correctly aligned with the collar of the box body in order for the snap-fit hooks to be inserted in the respective seats. Therefore, the solution described in EP3194287 B1 should facilitate closure of the container with cover. However, the solution described in EP3194287 B1 has the disadvantage that the force required to close the cover is excessive, i.e., an operator must force the cover to allow correct insertion and activation of the snap-fit couplings in the relevant openings. Moreover, the solution described in EP3194287 B1 has the disadvantage that the angular snap-fit couplings do not significantly increase the resistance of the container if dropped.

### DESCRIPTION OF THE INVENTION

The object of the present invention is to provide an improved container with cover, which overcomes the drawbacks described above.

The object of the present invention is to provide a box body and a cover that cooperate with each other so as to form a container with cover that is closed in a simple and safe manner and which has increased resistance to impacts, in particular to being dropped.

According to the present invention, there is provided a container, a box body and a cover as set forth in the appended claims.

### BRIEF DESCRIPTION OF THE DRAWINGS

The invention will now be described with reference to the accompanying drawings, which illustrate a non-limiting example of embodiment thereof, wherein:
- Figs. 1 and 2 are top-down perspective views of a container with cover according to the present invention in two respective different operating configurations;
- Fig. 3 illustrates an enlargement of the box body during a step of alignment with the cover according to the present invention;
- Fig. 4 is a bottom-up perspective view of a cover according to the present invention;
- Fig. 5 is an enlargement of a longitudinal section of a container according to the present invention with the cover coupled to the box body;
- Fig. 6 is a top-down perspective view of a variant of a box body according to the present invention.

### PREFERRED EMBODIMENT OF THE INVENTION

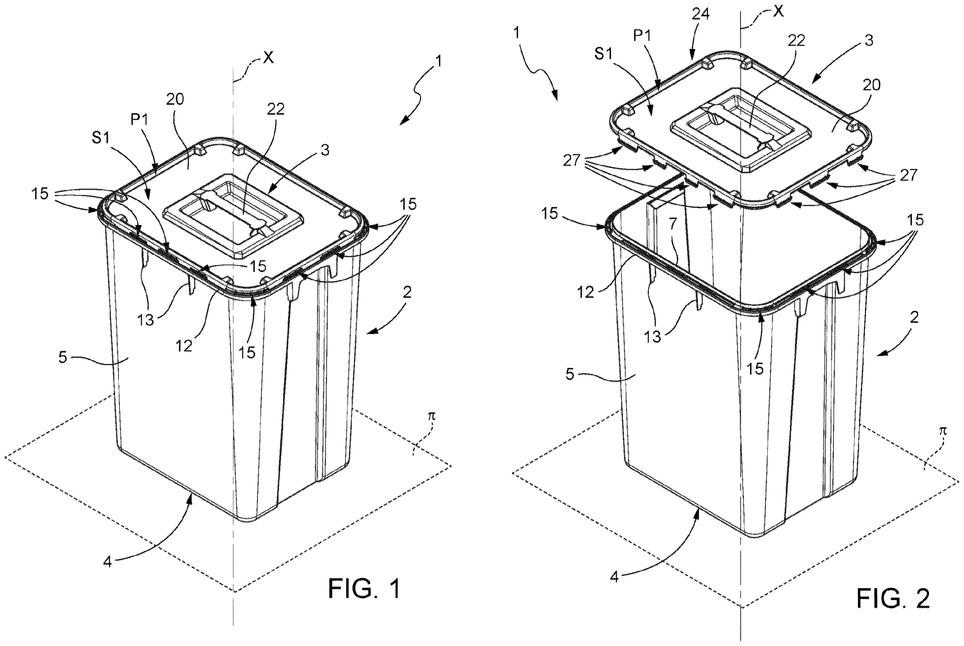

In Fig. 1, the reference number 1 indicates as a whole a container according to the present invention. The container 1 comprises, in turn, a box body 2 and a cover 3, which, in use, interlock with each other (as will be better illustrated below) so as to form a closed and indissoluble body.

The box body 3 has a parallelepiped cup-shaped body and extends along a longitudinal axis X. The box body 3 comprises a base 4 and a lateral wall 5 which protrudes from the base 4 along the longitudinal axis X so as to laterally border a housing 6. The base 4 is configured to be placed, in use, supported on a horizontal plane π. According to the example illustrated, the longitudinal axis X is substantially perpendicular to the plane π; hereunder terms with upper, lower, or similar, are used with reference to the box body 3 in a condition of normal support on the plane π.

The base 4 has a regular polygonal plan shape and, according to the example illustrated, the base has a plan shape that is substantially rectangular with joined corners. Without loss of generality, the shape and the size of the base 4 can differ from those illustrated, for example it can be square or of another shape.

Preferably, the lateral wall 5 is connected in correspondence of the lower end to the base 4 (in particular it is produced in one piece with the base, for example by means of an injection process of plastic materials). The lateral wall 2 is inclined outwards, i.e., it forms an angle greater than 90° with the base 4, so as to facilitate in use superimposing and producing stacks of a plurality of box bodies 2.

The upper end of the lateral wall 5 is bordered by a margin 7. The lateral wall 5 further comprises a collar 8 which externally protrudes from the lateral wall 5. In particular, the margin 7 is interposed, along the longitudinal axis X, between the collar 8 and upper end of the box body 2. The collar 8 surrounds the whole of the lateral wall 5. The collar 8 has, in plan view, substantially the shape of a rectangle with corners joined to each other. In particular, the collar 8 has, in plan view, four straight sides 10, identified hereunder with 10a, 10b, 10c, 10d. In particular, the sides 10a and 10c are opposite each other and of larger size with respect to the sides 10b and 10d, which are opposite each other and of smaller size. The straight sides 10 are connected to each other by corners 11, identified hereunder with 11I, 11II, 11III, 11IV. Each corner 11 is interposed and connects two respective sides 10 to each other. In particular, the corner 11I is interposed between the sides 10a and 10b; the corner 11II is interposed between the sides 10b and 10c; the corner 11III is interposed between the sides 10c and 10d; the corner 11IV is interposed between the sides 10d and 10a.

Advantageously, the collar 8 has a base wall 9 substantially parallel to the base 4, which protrudes from the lateral wall 5. The collar 8 further comprises a band 12 that surrounds the base wall 9 and is substantially parallel to the respective portion of the lateral wall 5. The band 12 protrudes along the longitudinal axis X both upwardly and downwardly from the base wall 9. The collar 8 further comprises a plurality of ribs 13 which externally protrude from the lateral wall 5 and downwardly from the base wall 9. The number and the distribution of the ribs 13 can vary with respect to those illustrated. The ribs 13 are configured to reinforce the collar 8 and prevent the base wall 9 from bending.

The collar 8 has a plurality of openings 14 that pass through the base wall 9. The number and the distribution of the openings 14 can vary with respect to those illustrated.

Advantageously, each corner 11 of the base wall 9 is without openings 14, in other words the base wall 9 in correspondence of each corner 11 is integral.

Advantageously, the collar 8 comprises a projecting part 15 placed in correspondence of each corner 11. According to a variant, not illustrated, the collar 8 comprises a single projecting part 15 placed in correspondence of a single corner 11; alternatively, the collar 8 comprises two projecting parts 15 disposed in correspondence of two opposite corners 11 (for example 11I, 11III or 11II, 11IV).

Each projecting part 15 has an abutment wall 16, which protrudes upwardly from the base wall 9. The abutment wall 16 is curved. In particular, the abutment wall 16 is structured so as to substantially follow the profile of the respective corner 11. The abutment wall 16 is disposed at a determinate distance d1 from the margin 7. According to the example illustrated, each abutment wall 16 is formed by the joining of a plurality of segments 18 adjacent to each other and inclined with respect to each other so as to follow the profile of the respective corner 11. The number, size and inclination of the segments 18 are variable. According to the example illustrated, the segments 18 have a curvature substantially concentric to the respective corner 11.

Advantageously, each abutment wall 16 comprises, in turn, ribs 19. In particular, each rib 19 is substantially a rib that extends on a plane perpendicular to the base 4. Advantageously, each abutment wall 16 comprises a plurality of ribs 19 distributed along its extension. In particular, each abutment wall 16 comprises two ribs 19 in correspondence of the ends and a plurality of intermediate ribs 19.

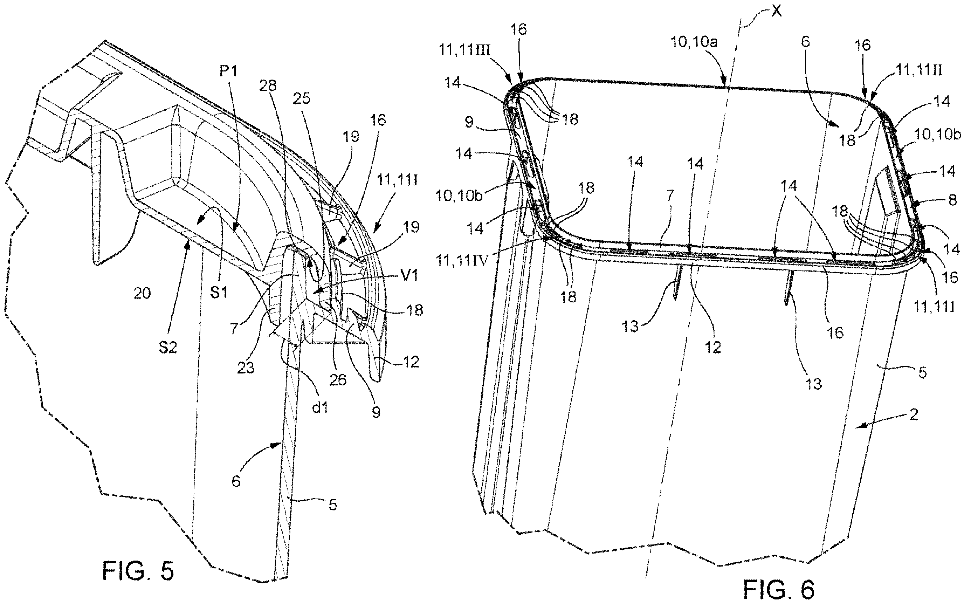

According to the example illustrated in Figs. 1 and 2, the collar 8 has one or more projecting parts 15 also distributed along the straight sides 10. In particular, the projecting parts 15 and the openings 14 are alternated with each other, so as to have a projecting part 15 interposed between two adjacent openings 14. Along the straight sides 10 the abutment walls 16 are rectilinear. In other words, the abutment walls 16 follow the profile of the respective portion of the base wall 9. The extension of the abutment walls 16 is variable. According to the variant illustrated in Fig. 6, the projecting parts 15 are present only in correspondence of the corners 11.

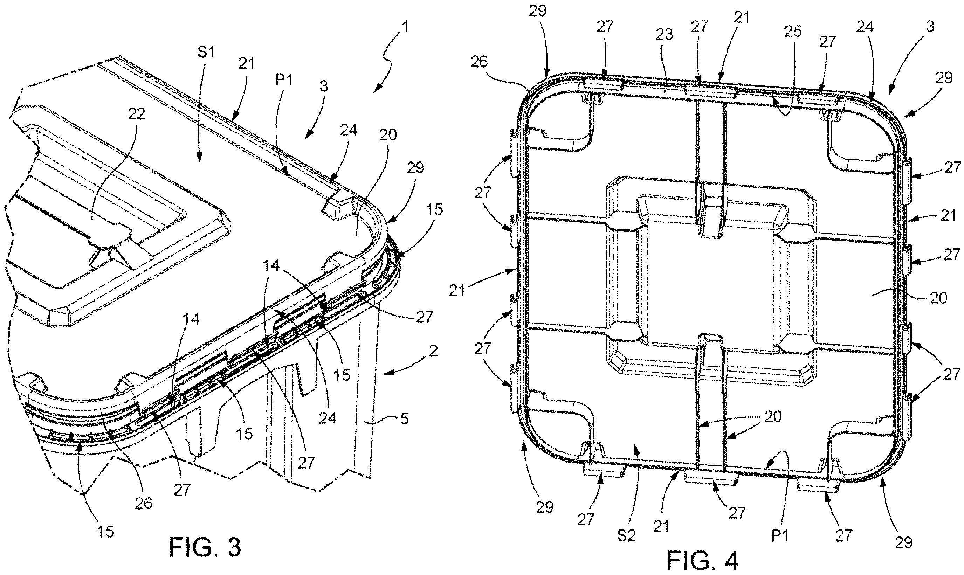

Fig. 4 illustrates a detail of the cover 3. In particular, the cover 3 has been produced from a substantially flat wall 20 having: a perimeter p1, an upper surface s1 (which faces the outside of the container 1 when it is closed) and a lower surface s2 (which faces the base 4 of the container 1 when it is closed) . The upper surface s1 and the lower surface s2 are opposite each other. The perimeter p1 of the wall 20 has a shape complementary to the shape of the margin 7 of the box body 2. According to the example illustrated, the perimeter p1 is rectangular in shape with rounded corners and the cover 3 comprises straight sides 21 and corners 29 that connect two adjacent straight sides 21.

In a manner that is known and illustrated schematically, the cover 3 has stiffening ribs and/or bosses. The cover 3 can comprise a handle 22 which protrudes from the upper surface s1 and can be held by a person.

Advantageously, the cover 3 comprises an inner wall 23 which extends substantially along the perimeter p1 and protrudes from the lower surface s2.

The inner wall 23 is substantially perpendicular to the wall 20 and is configured so as to be substantially parallel to the lateral wall 5, when the cover 3 is fixed to the box body 2.

The cover 3 further comprises a flap 24 which protrudes from the perimeter p1 and is bent in a U so as to border, laterally to the perimeter p1, a housing 25, in which the margin 7 of the box body 2 is housed, in use.

In particular, the flap 24 has an outer portion 26 the longitudinal extension of which is configured: so as to receive the margin 7 inside the housing 25; and to be able to be disposed abutting against the base wall 9 of the collar 8. In particular, the extension of the outer portion 26 of the flap 24 is greater, crosswise to the wall 20, to the extension along the longitudinal axis X of the margin 7 of the box body 2. Preferably, the flap 24 is inclined towards the outside of the box body 2.

Advantageously, the cover 3 comprises a plurality of snap-fit hooks 27 which protrude downwardly from the flap 24. In particular, each snap-fit hook 27 is bent outwards in a U shape. The concavity of the U of the snap-fit hooks 27 is facing upwards. The snap-fit hooks 27 are uniformly distributed along the straight sides 10 of the cover 3. Each snap-fit hook 27 is configured to form a shape and/or interference coupling with a respective opening 14 of the collar 8. In particular, each snap-fit hook 27 is configured to be inserted inside a respective opening 14 and form a bayonet type connection between the cover 3 and the collar 8 of the box body 2. In this way, the container 1 is closed in an indissoluble way.

Advantageously, the extension of the abutment wall 16 along the longitudinal axis X is such as to permit the flap 24 to engage/come into contact with the respective abutment wall 16 before each snap-fit hook 27 engages a respective opening 14.

Advantageously, the cover 3 has a seal 28 (schematized in Fig. 5), or strip of elastic material, disposed inside the housing 25 so as to be placed, in use, abutting against at least the upper end of the margin 7. In this way, advantageously, the container 1 is sealed and also prevents the escape of gases from the container 1 once it is closed.

Advantageously, the cover 3 has no snap-fit hooks in correspondence of the corners 29. In other words, only the flap 24 is present in correspondence of the corners 29. Advantageously, the flap 24 is configured to be housed, in use, inside the volume V1 (Fig. 5) defined by the distance d1 on the collar between the margin 7 and the abutment wall 16 of the projecting part 15. Advantageously, the flap 24 is inclined outwardly so as to be able to inserted inside the volume V1 only by deforming elastically. In this way, advantageously, the flap 24 is held pressed against the respective projecting part 15 so as to form an interference coupling.

In the case of the solution illustrated in Figs. 1 to 3, the flap 24 is also coupled by interference along the straight sides 21 in correspondence of respective projecting parts 15, in a similar manner to that illustrated above.

In use, when the container 1 must be closed, the cover 3 is fitted to the box body 2 so that the snap-fit hooks 27 are aligned with the respective openings 14, while the flap 24 is aligned with the projecting parts 15 regardless of the distribution of these projecting parts 15 along the perimeter p1, provided that the projecting parts 15 keep a substantially constant distance d1 between the respective abutment wall 16 and the margin 7.

By pushing the cover 3 against the box body 2, the flap 24 is pushed inside the volume V1 against the abutment walls 16 of all the projecting parts 15, so as to form the respective interference couplings. Moreover, each snap-fit hook 27 is inserted inside a respective opening 14 so as to form the respective shape and interference coupling.

Advantageously, with the solution described above it is possible to obtain a container 1 closed in an indissoluble way applying less force with respect to prior art solutions, such as EP3194287 B1. In fact, the insertion of a snap-fit hook 27 generates a greater resistance force with respect to the action of inserting the flap 24 inside the volume V1. Moreover, the solution of the type described above allows the operations to close the cover 3 to be simplified; in fact, interference coupling between the flap 24 and the abutment wall 16 before insertion of the snap-fit hooks 27 into the respective openings 14 allows small relative movements between the box body 2 and the cover 3 which facilitate the operations to correctly align the snap-fit hooks 27 with the openings 14.

Advantageously, the solution described above has a greater resistance to being dropped with respect to prior art solutions; in fact, the presence of the projecting parts 15 in correspondence of the corners 11 allows pre-tensioning to be applied between the box body 2 and the cover 3 as a function of the elastic deformation of the flap 24. The extent and the direction of application of the pre-tensioning is a function of the size of the flap 24 and of the projecting parts 15.

Advantageously, the presence of the ribs 19 prevents deformation of the respective abutment wall 16, increasing the solidity and strength of the box body 2.

Moreover, as the collar 8 is integral in correspondence of the corners 11, the mechanical strength of the box body 2 is greater with respect to prior art solutions. In fact, the openings in correspondence of the corners, as known, for example, from EP3194287 B1, produce a localized weakening of the collar with greater risk of breaking in case of impacts or being dropped.

## Claims

1. A container, in particular with a permanent closure, comprising a box body (2) and a cover (3), which are configured to interlock with each other in an indissoluble way; wherein, said box body (2) comprises a base (4) and a lateral wall (5) that define a cup-shaped body having a housing (6) accessible through an opening defined by an upper margin (7) of said lateral wall (5); wherein, the box body (2) comprises a collar (8) which externally protrudes from the lateral wall (5); the collar (8) having a polygonal plan shape with straight sides (10) and corners (11), each of which connects two adjacent straight sides (10); wherein, the collar (8) has integral corners (11), in other words corners are not provided with through openings; wherein, the cover (3) comprises a flat wall (20) bordered by a perimeter (p1) complementary to the shape of said margin (7); wherein the cover (3) comprises a flap (24), which protrudes outside said perimeter (p1) and has a U shape having a concavity facing, in use, the base (4) of the box body (2); wherein the collar (8) of the box body (2) comprises a projecting part (15), in particular disposed in correspondence of a respective corner (11); wherein the projecting part (15) and the flap (24) are configured to form an interference coupling.

2. A container according to claim 1, wherein the collar (8) comprises a plurality of projecting parts (15), in particular a projecting part (15) for each of two opposing corners (11) and/or a projecting part (15) for each corner (11).

3. A container according to claim 1 o 2, wherein the collar (8) comprises a base wall (9), which perpendicularly protrudes from the lateral wall (5) of the box body (2); wherein, each projecting part (15) comprises an abutment wall (16) which perpendicularly protrudes from base wall (9), in particular upwards; wherein, each abutment wall (16) follows the shape of the respective portion of the base wall (9); wherein, each abutment wall (16) is disposed at a determinate distance (d1) from the margin (7) so as to delimit a volume (V1) configured to house, at least partially, a respective portion (26) of said flap (24).

4. A container according to claim 3, wherein each abutment wall (16) comprises a plurality of segments (18) inclined with respect to each other.

5. A container according to claim 3 or 4, wherein the collar (8) comprises a plurality of ribs (19) distributed along a respective abutment wall (16) and configured to keep the respective portion of the abutment wall (16) perpendicular to the base wall (9).

6. A container according to any one of the preceding claims and comprising one or more projecting parts (15) along one or more straight sides of said collar (8).

7. A container according to any one of the preceding claims, wherein the cover (3) comprises a plurality of snap-fit hooks (27), which protrude downwardly from the straight sides (21) of said flap (24); wherein said collar (8) has a plurality of through openings (14) distributed along respective straight sides (10); wherein, the shape and the distribution of the snap-fit hooks (27) of the cover (3) are configured to form an interference coupling with the respective openings (14) of the base wall (9) of the collar (8).

8. A container according to claim 7, wherein the flap (24) of the cover (3) and each projecting part (15) of the box body (2) are configured to come into contact with each other before each snap-fit hook (27) engages a respective opening (14) of the collar (8).

9. A box body (2) for a container (1) according to any one of the preceding claims; wherein, said box body (2) comprises a base (4) and a lateral wall (5) that define a cup-shaped body having a housing (6) accessible through an opening defined by an upper margin (7) of said lateral wall (5); wherein, the box body (2) comprises a collar (8) which externally protrudes from the lateral wall (5); the collar (8) having a polygonal plan shape with straight sides (10) and corners (11), each of which connects two adjacent straight sides (10); wherein, the collar (8) has integral corners (11), in other words corners are not provided with through openings; wherein the collar (8) of the box body (2) comprises a projecting part (15), in particular disposed in correspondence of a respective corner (11); wherein the projecting part (15) is configured to form, in use, an interference coupling with a flap (24) of a cover (3).

10. A cover (3) for a container (1) according to any one of the preceding claims and comprising a flat wall (20) bordered by a perimeter (p1); wherein the cover (3) comprises a flap (24), which protrudes outside said perimeter (p1) and has a U shape having a concavity (6) facing, in use, the base (4) of the respective box body (2); wherein the flap (24) is configured to form, in use, an interference coupling with a projecting part (15) of respective box body(2).
